# FASCICULE DE BREVET EUROPEEN

(11) **EP 0 661 919 B1**
(45) Date de publication et mention de la délivrance du brevet: **05.12.2001**
(21) Numéro de dépôt: 93921965.5
(22) Date de dépôt: 28.09.1993
(51) Int. Cl.: A01H 15/00

(54) **Mycélium hétérocaryotique et homocaryon d'Agaricus bisporus ayant le caractère tétrasporée, leur utilisation et obtention**
Heterocaryotisches Mycelium und Homocaryon von Agaraicus bisporus, welches tetrasporalen Charakter aufweist, deren Verwendung und Herstellungsverfahren
Heterocaryotic mycelium and homocaryon of Agaricus bisporus, which has a tetrasporic character, their utilisation and process

(30) Priorité: 28.09.1992 FR 9211536
(43) Date de publication de la demande: 12.07.1995
(73) Titulaire: C.T.C.- CENTRE TECHNIQUE DU CHAMPIGNON, 49400 Distre (FR); INSTITUT NATIONAL DE LA RECHERCHE AGRONOMIQUE (INRA), 75007 Paris Cédex 07 (FR)
(72) Inventeur: CALLAC, Philippe, F-33140 Villenave d'Ornon (FR); IMBERNON, Micheline, F-33400 Talence (FR); BILLETTE, Christophe, F-33640 Castres (FR)
(74) Mandataire: Martin, Jean-Jacques
(86) Numéro de dépôt international: FR9300947
(87) Numéro de publication internationale: WO9407357

(56) Documents cités:
- DE-A- 3 428 988
- US-A- 4 996 390
- VAN GRIENSVEN -ED- 'The cultivation of mushrooms' 1988 , MUSHROOM EXPERIMENTAL STATION , HORST NL cité dans la demande Chapter 5, pages 101-123, G.FRITSCHE & A.S.M.SONNENBERG : "Mushroom strains" voir page 106, ligne 19, alinéa 2.2 - page 121, ligne 1; figures 5.2,5.3,5.9
- PROCEEDINGS FIRST INTERNATIONAL SEMINAR MUSHROOM SCIENCE Horst NL may 1991 L.J.L.D.VAN GRIENSVEN -ed- "Genetics and breeding of Agaricus" 1991, PUDOC WAGENINGEN , cité dans la demande
- MUSHROOM SCIENCE vol. 8 , 1972 pages 295 - 303 S.F.SONG ET AL 'Observations on the spored-basidium in the cultuvated mushroom (Agaricus bisporus)' cité dans la demande
- THEORETICAL APPLIED GENETICS vol. 70 , 1985 pages 52 - 56 R.DICKHARDT 'Homokaryotization of Agaricus bitorquis (Quel.)Sacc. and Agaricus bisporus (Lange)Imb.'
- APPLIED AND ENVIRONMENTAL MICROBIOLOGY vol. 54, no. 7 , Juillet 1988 pages 1643 - 1648 A.J.CASTLE ET AL 'Crosses among homokaryons from commercial and wild-collected strains of the mushroom Agaricus brunnescens (=A.bisporus)'

## Description

La présente invention a pour objet de nouvelles souches hybrides d'Agaricus bisporus dont les fructifications présentent un caractère majoritairement tétrasporé et/ou trisporé dans les conditions standard de culture, et permettent d'obtenir des homocaryons en grande quantité.

L'invention concerne également un procédé de préparation de telles souches hybrides et/ou l'utilisation de ces souches hybrides dans un procédé d'obtention et de sélection de souches d'Agaricus bisporus améliorées.

L'invention concerne en outre l'utilisation de tels hybrides à fructifications majoritairements tétrasporées et/ou trisporées dans l'obtention de quantités notables d'homocaryons et/ou dans un procédé d'obtention d'une souche d'Agaricus bisporée améliorée par croisement(s) et sélection.

Dans la présente demande, lorsqu'on parle d'une "souche", sans autre précision, il s'agit d'une souche d'Agaricus bisporus ou d'une souche interfertile avec Agaricus bisporus. Par ailleurs, l'expression "tétrasporé" sans autre précision, peut signifier, par exemple, "majoritairement tétrasporé et/ou trisporé, et faiblement bisporé", en particulier lorsqu'il est évident que le but recherché est de pouvoir disposer d'un grand nombre d'homocaryons. L'expression "à basides majoritairement tétrasporées" peut, pour des raisons analogues, signifier aussi bien : "dont les spores sont majoritairement homocaryotiques".L'expression "spore homocaryotique" désigne ici des spores dont la germination fournit des mycéliums homocaryotique (ou homocaryons).

On sait que les espèces du genre Agaricus présentent des basides normalement tétrasporées. Le champignon cultivé (ou champignon de couche) représente une exception notable, avec des basides majoritairement bisporées (généralement dans une proportion au moins égale à 90%). De là résulte l'appellation de cette espèce : Agaricus bisporus.

Les spores d'Agaricus bisporus contiennent normalement deux noyaux non frères issus de la méiose et leur germination en culture monospore conduit à l'obtention de myceliums hétérocaryotiques fructifères, c'est-à-dire capables de fructifier sans confrontation avec un mycélium compatible.

Agaricus bisporus est donc une espèce bisporée homothallique secondaire, selon la définition de J.R. RAPER, "Genetics of Sexuality in Higher Fungi", The Remaid Press Cy, New York, 1966. Sa sexualité est de type bipolaire, ce qui peut être exprimé par le fait que chacun des mycéliums issus d'une des quatre spores d'une des rares basides tétrasporées est compatible avec deux des mycéliums issus des trois autres spores.

Compte tenu de son caractère homothallique, il est difficile d'obtenir de grandes quantités d'homocaryons d'Agaricus bisporus qui seraient nécessaires pour réaliser des plans de sélection par croisements.

C'est en partie pour ces raisons que la variabilité génétique parmi les souches traditionnellement cultivées est actuellement restreinte à six classes génotypiques, deux de ces classes présentant le caractère blanc provenant de mutants apparus en culture, l'un en 1926, l'autre en 1960. Une septième classe dite "hybride" résulte d'un croisement effectué entre les deux souches blanches.

Par ailleurs, il existe des souches sauvages d'Agaricus bisporus, mais ces souches présentent un caractère brun dominant qui rend difficile leur utilisation dans la réalisation d'hybrides.

Sur toutes ces questions, on peut citer par exemple G.Fritsche et A.S.M. Sonnenberg, "The cultivation of Mushrooms" Van Griensven Ed. (1988), pp.101-123.

Les méthodes existantes d'obtention d'homocaryons d'Agaricus bisporus sont difficiles à mettre en oeuvre et surtout ne fournissent que de faibles quantités d'homocaryons différents. Ces méthodes consistent soit à mettre en germination des spores et à isoler et repérer des myceliums provenant de spores des rares basides tétrasporées, soit à isoler par micromanipulation des spores issues de basides tétrasporées, soit encore à isoler des protoplastes par lyse enzymatique de la paroi des cellules plurinucléées hétérocaryotiques constituant le mycélium ; voir par exemple G.Fritsche et A.S.M. Sonnenberg, article cité ci-dessus.

On sait que pour repérer des myceliums homocaryotiques et les distinguer des myceliums hétérocaryotiques, on peut utiliser diverses méthodes, par exemple :
- l'observation de la croissance du mycelium, généralement moins vigoureuse chez les myceliums homocaryotiques ;
- les essais de confrontation avec des homocaryons possédant différents allèles d'incompatibilité ;
- les essais de fructification ;
- l'utilisation d'un ou de préférence plusieurs marqueurs biochimiques, notamment enzymatiques, en particulier des alloenzymes (May et Royse, Experimental Mycology, Vol. 6, pp 283-292, 1982 ;
- ou encore l'utilisation d'un ou plusieurs marqueurs moléculaire(per exemple marqueurs RFLP ou RAPD) ; voir par exemple "Genetics and Breeding of Agaricus", Proceedings of the First International Seminar on Mushroom Science, Horst (Pays-Bas), 14-17 Mai 1991, L.J.L.D. Van Griensven Ed, PUDOC Wageningen 1991, en particulier pp 62-72 (P.A. Horgen et al.) et pp 73-77 (R.S. Khush et al.).

Les marqueurs biochimiques et moléculaires peuvent être utilisés de deux façons : ou bien on sait que la souche-mère est hétérozygote, et par exemple la perte de l'hétérozygotie chez le mycelium testé montre qu'il doit être homocaryotique, ou bien on confronte un mycelium M supposé homocaryotique avec un homocaryon intercompatible porteur d'un allèle différent pour le marqueur étudié, et si le mycelium résultant est hétéroallélique, c'est que le mycelium M était bien homocaryotique, et dans ce dernier cas l'utilisation d'un seul marqueur est suffisante pour conclure.

On a maintenant découvert qu'il existe des souches d'Agaricus sauvages très faiblement bisporées, dont par exemple au moins 80% des basides ont plus de deux spores (notamment quatre spores), qui sont interfertiles avec les souches sauvages et les souches commerciales d'Agaricus bisporus, et dont les hybrides avec Agaricus bisporus sont majoritairement tétrasporés et/ou trisporés, sans prendre de mesures spéciales à cet effet. Autrement dit, ce caractère tétrasporé et faiblement bisporé est dominant dans une telle hybridation. En outre, lesdits hybrides sont eux-mêmes interfertiles avec Agaricus bisporus.

De telles souches sauvages peuvent être trouvées notamment en Californie, dans le désert Sonoran. Une de ces souches est distribuée sous le n°ARP 61 par l'Agaricus Recovery Program (en abrégé : ARP) Post Office Box 461, Worthington PA 16262, USA.

D'autres souches analogues faiblement bisporées, telles que l'ARP 15 (également appelée "JB2" et déposée à l'ATCC sous le n°76072) et éventuellement certaines souches choisies parmi les souches ARP 116 à 167 peuvent également être acquises auprès de l'ARP.

En raison du fait que le phénotype tétrasporé de ces souches sauvages est, de façon surprenante, transmissible aux produits d'hybridation de façon dominante, il est possible d'utiliser de telles souches sauvages, dont les fructifications portent des spores en majorité homocaryotiques, pour réaliser des croisements avec des souches d'Agaricus bisporus conduisant à des hybrides dont le caractère tétrasporé et/ou trisporé (une majorité de spores est donc homocaryotique) permet d'obtenir, pour la première fois en quantités notables, des homocaryons dont on sait qu'ils sont nécessaires pour la réalisation ultérieure de croisements par hybridation en vue d'obtenir par sélection des variétés hybrides améliorées.

Il faut rappeler que les rares cas connus de souches dont un nombre important de basides portent plus de deux spores résultaient des conditions particulières (environnement) de la culture et non d'un caractère génétiquement transmissible ; voir par exemple J.Pelham, Mushroom Science 7, pp.49-64 (1965) et S.F. Song et al., Mushroom Science 8, 295-303 (1972). Il convient de noter ici que chez les souches tétrasporées utilisées et/ou obtenues selon la présente invention, le caractère tétrasporé est transmissible et est observé dans les conditions standard de culture, qui peuvent être par exemple les suivantes : compost à base de fumier de cheval et de paille ; étape d'envahissement du compost par le mycelium : 24-25°C ; terre de gobetage : mélange de tuffeau et de tourbe ; température après gobetage : 15-18°C ; humidité relative 85-95%.

L'invention permet donc de développer des schémas de sélection classiques qui n'étaient applicables auparavant qu'aux seules souches de champignons hétérothalliques. Les méthodes d'interfécondation contrôlée sont considérablement facilitées et les méthodes d'interfécondation libre sont rendues possibles. Des méthodes de mutagénèse sur spores de souches tétrasporées et éventuellement des méthodes de tri sur fructifications homocaryotiques peuvent aussi être envisagées.

On donne ci-après quelques exemples permettant de mieux comprendre l'intérêt de l'invention.

Soit une souche X d'Agaricus bisporus, bisporée. On peut isoler, selon les méthodes classiques rappelées ci-dessus, un homocaryon. Cet homocaryon de X est confronté avec un homocaryon compatible provenant d'une souche interfertile tétrasporée Y, telle que ARP 61, ou telle qu'une souche hybride tétrasporée issue de ARP 61, une telle souche permettant de disposer facilement de nombreux homocaryons. On obtient ainsi une souche hybride XY dont les fructifications seront majoritairement tétrasporées, et dont il sera possible d'obtenir de nombreux homocaryons puisque la grande majorité des spores formées par les fructifications d'une telle souche XY sont homocaryotiques. Dans la souche hybride XY, de même que dans les spores et les homocaryons qui en sont issus, la moitié du génome provient de la souche X. Par une série de rétrocroisements (ou "backcrosses") avec X, on peut incorporer chez les hybrides obtenus une proportion croissante du génome de X. Grâce aux hybrides tétrasporés obtenus à chaque rétrocroisement, on peut obtenir finalement en grande quantité des homocaryons dans lesquels on a incorporé au moins un caractère intéressant d'une souche X connue bisporée.

Grâce à l'invention, il est même possible d'obtenir facilement des souches tétrasporées isogéniques, dans la proportion désirée (par exemple à plus de 90% ou à plus de 95%), d'une souche bisporée X d'Agaricus bisporus. On peut alors disposer notamment de grandes quantités d'homocaryons dont la plus grande partie du génome peut provenir de toute souche d'Agaricus bisporus (normalement bisporée) considérée comme intéressante, y compris de toute souche commerciale existante.

On peut en particulier isoler et identifier deux mycéliums homocaryotiques intercompatibles X1 et X2, issus d'une souche X d'Agaricus bisporus bisporée, par exemple par la méthode des protoplastes. On confronte l'un des deux homocaryons, par exemple X1, avec un homocaryon d'une souche Y interfertile tétrasporée, par exemple telle que définie précédemment. On obtient une souche hybride XY dont les fructifications fournissent un fort pourcentage de basides tétrasporées et/ou trisporées. On peut alors obtenir facilement des homocaryons de XY dont la moitié du génome provient de la souche X. Par une série de rétrocroisements avec la souche X, en utilisant à chaque fois l'homocaryon X1 comme partenaire de croisement, et en sélectionnant à chaque étape les hybrides tétrasporés, on peut obtenir successivement des hybrides, notamment tétrasporés, dont des proportions croissantes du génome proviennent de X, ou plus précisément de X1. En théorie, comme il est facile de le déterminer, ces proportions sont de l'ordre de 75%, 87,5%, 93,75% et 96,9% après 1, 2, 3 ou 4 rétrocroisements, respectivement.

Lorsque le taux d'isogénie souhaité est atteint, on peut même confronter un homocaryon du dernier hybride tétrasporé issu des rétrocroisements, avec l'homocaryon X2 dont on sait qu'il est compatible avec X1. On obtient ainsi des hybrides X' dont une proportion importante du génome provient de X et qui possèdent en outre l'hétérozygotie caractéristique de X grâce au croisement final avec X2.

Un hybride isogénique X' tétrasporé pourra être la source d'un grand nombre d'homocaryons permettant le croisement de la souche X avec d'autres souches Z intéressantes, notamment à l'aide d'homocaryons de souches Z' rendues tétrasporées et rendues au moins partiellement isogéniques de Z, par la méthode qui vient d'être exposée. La souche Z est par exemple une souche provenant d'un site éloigné du site d'origine de la souche X (ou des parents de X). On peut ainsi faire de nombreux essais de croisement de X' et Z ou Z', en particulier dans le but de tirer parti de la vigueur généralement caractéristique des hybrides issus de parents génétiquement éloignés.

L'un des avantages obtenus grâce au fait que les homocaryons deviennent facilement disponibles en grandes quantités, est que l'on peut opérer sur un grand nombre d'homocaryons choisis au hasard, sans le risque d'une contre-sélection inhérent au petit nombre d'homocaryons obtenus par les voies classiques. Toutefois, lors des différents rétrocroisements conduisant aux souches tétrasporées au moins partiellement isogéniques de X (ou de Z), on peut en outre trier, parmi les homocaryons, ceux dont les performances, lors de croisements avec le partenaire d'hybridation finale (Z dans le cas de X), sont en moyenne satisfaisantes ou même, le cas échéant, très intéressantes.

Il devient également facile, grâce à l'invention, d'introduire un caractère récessif intéressant d'une souche bisporée à une autre. En effet, soit par exemple un caractère intéressant monogénique récessif a, présent dans une souche bisporée Xaa(b), homozygote pour l'allèle récessif a. L'indication (b) désigne ici le phénotype bisporé. On souhaite transférer a dans une souche bisporée ZAA(b) homozygote pour l'allèle dominant A. Pour cela, on peut procéder de la façon suivante. Dans un premier temps, on réalise un hybride XYAa(T) entre un homocaryon a(b) de X obtenu par voie classique (tri de myceliums issus de spores ou de protoplastes) et un homocaryon A(T) provenant de la germination d'une spore d'une souche tétrasporée YAA(T). L'indication (T) désigne ici le phénotype tétrasporé dominant. Cet hybride XYAa(T) est tétrasporé et ses spores produisent de nombreux homocaryons dont certains sont de type a(T), c'est-à-dire portent à la fois l'allèle a et le caractère tétrasporé. Ces homocaryons a(T) sont repérables car ce sont les seuls qui, rétrocroisés avec l'homocaryon a(b) de X, donnent des hybrides à la fois tétrasporés et présentent le caractère a souhaité. Dans un deuxième temps, on introduit par rétrocroisements l'allèle a porté par l'un des homocaryons a(T), dans la souche ZAA(b). Pour cela, on peut d'abord obtenir, par exemple par la méthode des protoplastes, deux homocaryons intercompatibles Z1A(b) et Z2A(b). En croisant Z1A(b) avec un homocaryon a(T) issu de XYAa(T), on peut obtenir un hybride tétrasporé, et parmi les homocaryons issus des spores de cet hybride, on peut de nouveau repérer, comme indiqué précédemment, ceux qui sont a(T), c'est-à-dire porteurs de l'allèle a et du caractère tétrasporé. Ces nouveaux homocaryons a(T) sont rétrocroisés plusieurs fois de suite avec Z1A(b) jusqu'à obtenir un homocaryon Z'1a(T) fortement isogénique à Z1A(b), mais portant l'allèle a et le caractère tétrasporé.

Les mêmes types de croisements sont effectués parallèlement à partir de Z2A(b) et d'un homocaryon a(T) issu de XYAa(T) pour aboutir à un homocaryon Z'2a(T) fortement isogénique à Z2A(b).

Les deux homocaryons Z'1a(T) et Z'2a(T) sont finalement croisés pour obtenir un hétérocaryon Z'aa(T) fortement isogénique à Z (en particulier l'hétérozygotie présente dans Z se retrouve dans Z') mais tétrasporé et présentant le caractère souhaité. Si désiré, les deux homocaryons Z'1 et Z'2 obtenus à l'issue de chaque série de rétrocroisements peuvent aussi être choisis porteurs du caractère bisporé. Dans ce cas, l'hybride final Z'aa(b) est bisporé.

Bien entendu, la méthode qui vient d'être décrite est applicable, avec les adaptations nécessaires, dans d'autres cas, y compris lorsque le caractère étudié est gouverné par deux à trois gènes à loci indépendants.

Lorsque le caractère étudié, ou le caractère que l'on désire incorporer dans une souche hybride, est gouverné par un nombre important de gènes, par exemple un nombre égal ou supérieur à 3, il est généralement plus facile de faire une sélection massale. Dans un tel cas, on pourra encore tirer facilement profit de la présente invention en opérant par exemple de la façon décrite ci-après.

On multiplie sur un substrat approprié un mycélium primaire obtenu à partir d'un homocaryon d'une souche mère intéressante X, et l'on ensemence ledit substrat portant ce mycélium primaire avec une pluralité de spores ou de mycéliums majoritairement homocaryotiques issus d'un champignon Y majoritairement tétrasporé et/ou trisporé interfertile avec Agaricus bisporus, Y étant par exemple tel que défini précédemment. On cultive alors les mycéliums secondaires obtenus, dans des conditions qui permettent la fructification, et on sélectionne les carpophores susceptibles de présenter le caractère recherché.

Les méthodes de sélection qui ont été exposées ci-dessus peuvent éventuellement être combinées entre elles.

Grâce à l'invention, on peut également étudier le déterminisme d'un caractère phénotypique donné, selon des méthodes classiques qui ne pouvaient pas être utilisées auparavant : par exemple hybridation par croisement de deux souches parentes, avec obtention d'une première génération d'hybrides dite F1, suivie soit de rétrocroisement avec une des souches parentes, soit de l'obtention d'une génération d'hybrides (dite F2) obtenue par croisement entre homocaryons, issus d'un hybride F1, et enfin étude des hybrides ainsi obtenus.

En outre, grâce à l'invention, qui permet d'obtenir des hybrides tétrasporés fournissant un grand nombre d'homocaryons, il est possible d'effectuer des opérations de mutagénèse, selon les méthodes connues, sur les spores homocaryotiques obtenues en grand nombre. Dans ce cas, l'ensemble du mycélium obtenu à partir de spores monocaryotiques mutés est mutant.

Si on peut obtenir des fructifications homocaryotiques (ce qui semble être possible chez Agaricus bisporus), on peut alors observer le caractère muté sur les carpophores, même s'il est récessif.

Les nouvelles souches d'Agaricus hybrides, obtenues par croisement(s), sont interfertiles avec Agaricus bisporus, et les fructifications se caractérisent par le fait qu'une faible proportion des basides qu'elles portent sont bisporées. Généralement les autres basides (non bisporées) sont tétrasporées et/ou trisporées. Il est admis que les spores issues de basides bisporées sont le plus souvent hétérocaryotiques, que les deux tiers des spores issues de basides trisporées sont homocaryotiques, et que la totalité des spores issues de basides tétrasporées sont homocaryotiques. Les souches hybrides de l'invention obtenues à la première génération (par croisement avec une souche telle que l'ARP 61) sont majoritairement tétrasporées, la proportion des basides bisporées étant inférieure à 8% et le plus souvent à 2%. Chez les hybrides obtenus lors des croisements ultérieurs, on peut observer un "type tétrasporé" dont moins de 15% (en général moins de 7%) des basides sont bisporées, les autres basides étant essentiellement tétrasporées et trisporées, et un "type bisporé" dont plus de 25% des basides (et en général plus de 45%) sont bisporées et moins de 15% (en général moins de 7%) sont tétrasporées. Bien entendu, de telles souches de type bisporé ne font pas partie de l'invention.

Une des caractéristiques remarquables des souches hybrides faiblement bisporées obtenues (moins de 15% de basides bisporées) est donc que la majorité des spores sont homocaryotiques. On peut calculer aisément que par exemple une souche contenant 15% de basides bisporées, 40% de basides tétrasporées et 45% de basides trisporées donnera en principe plus de 75% de spores homocaryotiques.

Les souches hybrides telles que définies précédemment, dont les fructifications portent des basides dont au moins 40% sont tétrasporées. Généralement, les autres basides (c'est-à-dire autres que bisporées et tétrasporées) ont au moins trois spores et sont le plus souvent trisporées. Bien entendu, on ne tient pas compte ici des rares basides erratiques monosporées.

Les souches telles que définies ci-dessus dont moins de 8% des basides sont bisporées, ainsi que les souches dont plus de 45%, et en particulier plus de 50% des basides sont tétrasporées.

L'invention a pour objet les formes homocaryotiques ou hétérocaryotiques de ces nouvelles souches hybrides, ainsi que les carpophores obtenus par fructification desdites souches. L'invention s'étend en particulier aux homocaryons et aux myceliums homocaryotiques obtenus notamment par germination des spores issues de ces carpophores, ou obtenus à partir de protoplastes.

Des caractères phénotypiques intéressants peuvent être incorporés dans la souche hybride en particulier ceux qui sont habituellement recherchés, dans les opérations de sélection, dans le domaine des champignons de couche, et notamment choisis parmi un ou plusieurs des caractères suivants : forme, taille et couleur (notamment couleur blanche) des carpophores, rendement, aptitude à fructifier dans des conditions et/ou sur un substrat donné, résistance à certaines maladies (virales, bactériennes ou fongiques) ou à certains produits de traitements, fructification en volées plus ou moins marquées, voire absentes, absence de rochers, type d'ancrage (par exemple solidité de l'ancrage) et/ou caractères organoleptiques, nutritionnels ou toxicologiques (par exemple faible taux d'agaritine et/ou de ses dérivés).

Les caractères génotypiques intéressants conférés à la souche hybride sont par exemple ceux résultant de l'incorporation d'une partie, ou d'une proportion importante, du génome d'une souche connue choisie pour ses propriétés particulières ou choisie pour son origine particulière (notamment de façon à exploiter, dans ce dernier cas, la vigueur des hybrides ainsi obtenus, comme déjà indiqué précédemment).

Les souches hybrides de l'invention peuvent donc être caractérisées par le fait qu'elles ont incorporé de tels caractères phénotypiques et/ou génotypiques.

L'invention concerne en particulier une souche hybride telle que définie précédemment, qui possède au moins un caractère phénotypique et/ou génotypique non présent chez les souches sauvages d'Agaricus, à basides majoritairement tétrasporés, interfertiles avec Agaricus bisporus.

Les souches hybrides selon l'invention sont notamment celles qui présentent au moins un caractère phénotypique et/ou génotypique intéressant présent dans une souche parentale bisporée ou dans une souche parentale issue d'une souche bisporée.

L'invention a également pour objet l'utilisation des homocargons ou des myceliums hétérocaryotique comme produit de départ pour l'obtention de souches d'Agaricus bisporus modifiées, bisporées ou non.

On peut mettre en oeuvre cette utilisation par exemple selon une ou plusieurs des méthodes suivantes :
- on effectue au moins un croisement, à l'aide d'homocaryons, de ladite souche hybride avec une souche d'Agaricus bisporus, et l'on sélectionne les produits de croisement obtenus, possédant un caractère phénotypique ou génotypique de ladite souche d'Agaricus bisporus ;
- ou on multiplie sur un substrat approprié un mycélium primaire obtenu à partir d'un homocaryon d'une souche d'Agaricus bisporus, et l'on ensemence ledit substrat portant ce mycélium primaire avec une pluralité de spores ou de mycéliums issus d'une souche hybride selon l'invention ;
- ou l'on effectue des opérations de mutagénèse sur les spores homocaryotiques d'une souche hybride selon l'invention ;
- ou l'on insère au moins un gêne ou fragment de gène dans le génome d'un homocaryon, d'un hétérocaryon ou d'un protoplaste issu d'une souche hybride selon l'invention.

Les exemples suivants illustrent l'invention.

### EXEMPLE 1

On a utilisé comme souche parentale la souche ARP 61.

Cette souche est brune. Environ 90% des basides observées sur les exemplaires obtenus en culture portent quatre spores ou plus, et 2% environ sont bisporées.

Son profil électrophorétique en alcool déshydrogénase (ADH) correspond à l'allèle 149 (selon D.J. Royse et May, Agric. Biol. Chem. 53(11), 2861-2866, 1989).

Cet allèle 149 est également présent dans des souches commerciales telles que Somycel 611 (Somycel) ou Royal 5A (Royal Champignon).

Son profil en estérase montre un allèle que l'on trouve également chez la souche canadienne Ag 89 (réf. : Agaricus Genetics Research Collection, Erindale College, Université de Toronto (Canada) ; souche décrite par D.Mallock, Mycologia 79(6) pp.839-846, 1987), et dans des souches commerciales telles que C45 (Le Lion).

274 cultures monospores ont été obtenues à partir des spores de ARP 61. Les confrontations intra-souche ont permis de montrer que plus de 90% de ces cultures monospores sont des homocaryons et que la souche est bipolaire.

Les confrontations intersouches entre d'une part la souche tétrasporée ARP 61 et d'autre part des souches d'Agaricus bisporus sauvages, américaines ou françaises, ou des souches commerciales, ont permis d'établir les résultats suivants :
- les homocaryons issus de la souche ARP 61 sont intercompatibles avec tous les homocaryons avec lesquels ils ont été confrontés, provenant de diverses souches d'Agaricus bisporus sauvages françaises, de la souche sauvage américaine Wc 240 (Pennsylvania State University Mushroom Culture Collection, souche décrite par May et Royse, Mushroom Science, N°XI, pp799-817, Australie 1981), et des souches commerciales U1 (Somycel), X1 (Le Lion), 30A (Royal Champignon), 191 (Somycel).

Dans tous les cas, des souches hybrides ont été isolées, qui présentent un profil hétérozygote en ADH alors que tous les homocaryons utilisés pour ces croisements présentent un profil à une seule bande.

Cela confirme le caractére hétérocaryotique des hybrides.

Les souches hybrides ont été mises en culture et ont, pour la plupart fructifié.

Dans tous les cas étudiés, sauf un, tous les carpophores hybrides présentent plus de 50% (généralement entre 65 et 85%) de basides à quatre spores ou plus, et moins de 8% de basides à deux spores. Les spores mises en cultures germent normalement. L'interfertilité entre la souche ARP 61 tétrasporée et les souches classiquement bisporées a donc été démontrée.

La souche ARP 61 peut être considérée comme appartenant à l'espèce Agaricus bisporus. Le caractère tétrasporé est de plus dominant sur le caractère bisporé.

### EXEMPLE 2

Des croisements ont été effectués entre des homocaryons de ARP 61 et des homocaryons provenant d'un protoplaste de la souche commerciale U1 qui donne des fructifications blanches. Chez les hybrides obtenus à la première génération, sur 43 hybrides étudiés, tous sont de caractère tétrasporé (moins de 7%, et en général moins de 2% de basides bisporées et plus de 50%, et en général plus de 60%, de basides tétrasporées), à l'exception d'un hybride qui était plutôt de type bisporé, cette exception étant due à une contamination accidentelle. Les homocaryons issus des spores d'un des hybrides obtenus ont été croisés avec un second homocaryon provenant d'un protoplaste de U1 et intercompatible avec le premier homocaryon de U1. Le génome des hybrides obtenus après ce rétrocroisement provient donc pour 75% du génome de U1 et pour 25% du génome de ARP 61.

Pour ce qui concerne le nombre de spores, sur 39 hybrides, obtenus après rétrocroisement, qui ont été étudiés, 22 sont de type bisporé (plus de 25%, et en général plus de 45%, de basides bisporées et moins de 15%, en général moins de 7%, de basides tétrasporées), et 17 sont de type tétrasporé (moins de 15% et en général moins de 7% de basides bisporées, et plus de 40%, en général plus de 45%, de basides tétrasporées).

Sur les 39 hybrides étudiés (21 à fructification blanche et 18 à fructification brune), plus d'un quart donnent des fructifications à la fois blanches et de type tétrasporé.

On a remarqué que le caractère tétrasporé est généralement transmis avec le caractère sexuel, ce qui se traduit par le fait que les homocaryons, issus de l'hybride, qui sont porteurs dudit caractère, sont généralement compatibles avec l'homocaryon parental non porteur de ce caractère. Un simple test de compatibilité permet donc de sélectionner les homocaryons issus des spores de l'hybride qui sont capables de transmettre le caractère tétrasporé (ou plus généralement le caractère "à fructifications dont les spores sont majoritairement homocaryotiques").

## Revendications

1. Mycélium hétérocaryotique isogénique à plus de 75% d'une souche X donnée d'Agaricus bisporus bisporée, ledit mycélium ayant le caractère tétrasporé, c'est-à-dire donnant des fructifications dont les spores sont majoritairement homocaryotiques.

2. Homocaryon susceptible d'être obtenu à partir de spores provenant de fructifications d'un mycélium hétérocaryotique selon la revendication 1, ledit homocaryon portant le caractère tétrasporé.

3. Mycélium hétérocaryotique selon la revendication 1 ayant, pour la partie isogénique, au moins une partie de l'hétérozygotie caractéristique de ladite souche X.

4. Homocaryon susceptible d'être obtenu à partir de spores provenant de fructifications d'un mycélium hétérocaryotique selon la revendication 3, ledit homocaryon portant le caractère tétrasporé.

5. Mycélium hétérocaryotique isogénique à plus de 50% d'une souche X donnée d'Agaricus bisporus bisporée, ledit mycélium ayant le caractère tétrasporé, c'est-à-dire donnant des fructifications dont les spores sont majoritairement homocaryotiques, et ayant, pour la partie isogénique, au moins une partie de l'hétérozygotie caractéristique de ladite souche X.

6. Homocaryon susceptible d'être obtenu à partir de spores provenant de fructifications d'un mycélium hétérocaryotique selon la revendication 5, ledit homocaryon portant le caractère tétrasporé.

7. Mycélium hétérocaryotique isogénique à plus de 50% d'une souche donnée Z d'Agaricus bisporus bisporée, ledit mycélium ayant le caractère tétrasporé et étant homozygote pour un caractère récessif absent dans ladite souche Z.

8. Mycélium hétérocaryotique selon la revendication 7, isogénique à plus de 75% de ladite souche Z.

9. Mycélium hétérocaryotique selon la revendication 7 ou 8, possédant le caractère tétrasporé.

10. Mycélium hétérocaryotique isogénique à plus de 50% d'une souche donnée Z d'Agaricus bisporus bisporée, ledit mycélium ayant le caractère tétrasporé et comportant un allèle dominant et un allèle nécessif d'un caractère dont l'allèle nécessif est absent dans la souche Z.

11. Homocaryon susceptible d'être obtenu à partir de spores provenant de fructifications d'un mycélium hétérocaryotique tel que défini dans la revendication 9 ou 10.

12. Homocaryon selon la revendication 11, portant ledit caractère récessif et le caractère tétrasporé.

13. Homocaryon selon la revendication 11, portant ledit caractère récessif et le caractère bisporé.

14. Utilisation d'au moins un homocaryon tel que défini dans la revendication 2, 4 ou 6 dans l'obtention d'hybrides dérivés de ladite souche X.

15. Utilisation d'au moins un homocaryon tel que défini dans la revendication 12 ou 13 dans l'obtention d'hybrides dérivés de la souche Z, lesdits hybrides étant homozygotes pour un caractère récessif absent de la souche Z.

16. Utilisation d'un mycélium hétérocaryotique tel que défini dans l'une quelconque des revendications 1, 3, 5, 7, 9 et 10, dans l'obtention de carpophores.

17. Procédé d'obtention d'un mycélium hétérocaryotique tel que défini dans la revendication 1 ou 3, dans lequel :
- on effectue un croisement d'un premier homocaryon, issu de la souche X, avec un second homocaryon issu d'une souche Y d'Agaricus, pour obtenir un hybride XY, la souche Y étant interfertile avec Agaricus bisporus et ayant le caractère tétrasporé,
- et on soumet des homocaryons issus de spores homocaryotiques de l'hybride XY à un nombre suffisant de rétrocroisements avec ledit premier homocaryon pour incorporer une proportion suffisante du génome de X dans l'hybride final, en sélectionnant à chaque étape de rétrocroisement les hybrides ayant le caractère tétrasporé.

18. Procédé d'obtention d'un mycélium hétérocaryotique tel que défini dans la revendication 5, dans lequel :
a) on isole deux homocaryons intercompatibles X1 et X2 de la souche X,
b) on effectue un croisement de l'homocaryon X1 avec un homocaryon issu d'une souche Y d'Agaricus ayant le caractère tétrasporé et interfertile avec Agaricus bisporus, pour obtenir un hybride X1Y,
c) on soumet des homocaryons issus de spores homocaryotiques d'un hybride obtenu au stade b) à un nombre suffisant de rétrocroisements avec lesdits homocaryons issus de la souche X pour incorporer une proportion suffisante du génome de X dans l'hybride final, et dans lequel on effectue au moins un des rétrocroisements avec X2.

19. Procédé selon la revendication 18, dans lequel on effectue le croisement et des éventuels rétrocroisements avec l'homocaryon X1, à l'exception du dernier rétrocroisement qui est effectué avec l'homocaryon X2, étant entendu qu'à l'issue de chaque étape de croisement et de rétrocroisement, sauf éventuellement à l'issue de l'avant-dernier rétrocroisement on sélectionne des homocaryons portant le caractère tétrasporé.

20. Procédé d'obtention d'un mycélium hétérocaryotique tel que défini dans l'une quelconque des revendications 7 à 9, homozygote pour au moins un allèle d'un caractère récessif absent dans la souche Z, dans lequel :
a) on croise une souche X d'Agaricus bisporus bisporée, homozygote pour ledit allèle, avec une souche Y d'Agaricus ayant le caractère tétrasporé, pour obtenir un hybride XY,
b) on choisit un homocaryon H de XY portant à la fois l'allèle récessif et le caractère tétrasporé,
c) on choisit deux homocaryons intercompatibles Z1 et Z2 de la souche Z,
d) on effectue un croisement entre ledit homocaryon H et Z1, suivi éventuellement d'au moins un rétrocroisement avec Z1, en sélectionnant à chaque étape un homocaryon d'un hybride obtenu ayant le caractère tétrasporé, ledit homocaryon portant ledit allèle récessif,
e) on effectue un croisement entre ledit homocaryon H et Z2, suivi éventuellement d'au moins un rétrocroisement avec Z2, en sélectionnant à chaque étape un homocaryon d'un hybride obtenu dont les fructifications ont des spores ayant majoritairement le caractère tétrasporé, ledit homocaryon portant ledit allèle récessif,
f) et on recroise entre eux, à l'aide d'homocaryons portant ledit allèle récessif, les produits de croisement obtenus en d) et en e), respectivement.

21. Procédé selon la revendication 20, dans lequel on effectue l'étape f) à l'aide de deux homocaryons dont l'un au moins porte le caractère tétrasporé.

22. Procédé selon la revendication 20, dans lequel on effectue l'étape f) à l'aide de deux homocaryons portant le caractère bisporé.

23. Carpophore susceptible d'être obtenu par fructification d'un mycelium hétérocaryotique tel que défini dans l'une quelconque des revendications 1, 3, 5 et 7 à 10.

## Patentansprüche

1. Heterokaryotisches Mycelium, welches zu mehr als 75 % mit einem Stamm, genannt X, des bisporen Agaricus bisporus isogen ist, wobei das Mycelium tetrasporen Charakter hat, d. h., Fruchtentwicklungen bildet, bei denen die Sporen hauptsächlich homokaryotisch sind.

2. Homokaryon, welches ausgehend von Sporen erhalten werden kann, die aus den Fruchtentwicklungen eines heterokaryotischen Myceliums gemäß Anspruch 1 hervorgegangen sind, wobei das Homokaryon das tetraspore Merkmal trägt.

3. Heterokaryotisches Mycelium gemäß Anspruch 1, welches in dem isogenen Bereich wenigstens einen Heterozygotiebereich aufweist, der für den Stamm X charakteristisch ist.

4. Homokaryon, welches ausgehend von Sporen erhalten werden kann, die aus den Fruchtentwicklungen eines heterokaryotischen Myceliums gemäß Anspruch 3 hervorgegangen sind, wobei das Homokaryon das tetraspore Merkmal trägt.

5. Heterokaryotisches Mycelium, welches zu mehr als 50 % mit einem Stamm, genannt X, des bisporen Agaricus bisporus isogen ist, wobei das Mycelium tetrasporen Charakter hat, d. h., Fruchtentwicklungen bildet, bei denen die Sporen hauptsächlich homokaryotisch sind, und in dem isogenen Bereich wenigstens einen Heterozygotiebereich aufweist, der für den Stamm X charakteristisch ist.

6. Homokaryon, welches ausgehend von Sporen erhalten werden kann, die aus den Fruchtentwicklungen eines heterokaryotischen Myceliums gemäß Anspruch 5 hervorgegangen sind, wobei das Homokaryon das tetraspore Merkmal trägt.

7. Heterokaryotisches Mycelium, welches zu mehr als 50 % mit einem Stamm, genannt Z, des bisporen Agaricus bisporus isogen ist, wobei das Mycelium tetrasporen Charakter hat und für ein rezessives Merkmal, welches nicht in dem Stamm Z vorkommt, homozygot ist.

8. Heterokaryotisches Mycelium gemäß Anspruch 7, welches zu mehr als 75 % mit dem Stamm Z isogen ist.

9. Heterokaryotisches Mycelium gemäß Anspruch 7 oder 8, welches tetrasporen Charakter besitzt.

10. Heterokaryotisches Mycelium, welches zu mehr als 50 % mit einem Stamm, genannt Z, des bisporen Agaricus bisporus isogen ist, wobei das Mycelium tetrasporen Charakter hat und ein dominantes Allel und ein rezessives Allel eines Merkmals trägt, wobei das rezessive Allel in dem Stamm Z nicht vorkommt.

11. Homokaryon, welches ausgehend von Sporen erhalten werden kann, die aus den Fruchtentwicklungen eines heterokaryotischen Myceliums gemäß den Ansprüchen 9 oder 10 hervorgegangen sind.

12. Homokaryon gemäß Anspruch 11, welches das rezessive Merkmal und das tetraspore Merkmal trägt.

13. Homokaryon gemäß Anspruch 11, welches das rezessive Merkmal und das bispore Merkmal trägt.

14. Verwendung wenigstens eines Homokaryons gemäß Anspruch 2, 4 oder 6 zum Erhalt von Hybriden, die von dem Stamm X abstammen.

15. Verwendung wenigstens eines Homokaryons gemäß Anspruch 12 oder 13 zum Erhalt von Hybriden, die von dem Stamm Z abstammen, wobei die Hybriden homozygot für ein rezessives Merkmal sind, welches im Stamm Z nicht vorkommt.

16. Verwendung eines heterokaryotischen Myceliums nach wenigstens einem der Ansprüche 1, 3, 5, 7, 9 und 10 zum Erhalt von Carpophoren.

17. Verfahren zum Erhalt eines heterokaryotischen Myceliums gemäß Anspruch 1 oder 3, in dem man
- eine Kreuzung eines ersten Homokaryons, welches aus dem Stamm X hervorgegangen ist, mit einem zweiten Homokaryon, welches aus einem Stamm Y von Agaricus hervorgegangen ist, durchführt, um ein Hybrid XY zu erhalten, wobei der Stamm Y interfertil mit Agaricus bisporus ist und tetrasporen Charakter hat,
- und die aus den homokaryotischen Sporen des Hybrids XY hervorgegangenen Homokaryen einer ausreichenden Anzahl von Rückkreuzungen mit dem ersten Homokaryon unterwirft, um einen ausreichenden Anteil des Genoms von X in das entstehende Hybrid einzubauen, wobei in jeder Rückkreuzungsstufe die Hybriden mit tetrasporem Charakter ausgewählt werden.

18. Verfahren zum Erhalt eines heterokaryotischen Myceliums gemäß Anspruch 5, in dem man
a) zwei interkompatible Homokaryen X1 und X2 des Stammes X isoliert,
b) eine Kreuzung des Homokaryons X1 mit einem Homokaryon, welches aus einem Stamm Y von Agaricus hervorgegangen ist, welcher tetraspor und interfertil mit Agaricus bisporus ist, durchführt, um ein Hybrid X1Y zu erhalten,
c) und die aus den homokaryotischen Sporen eines in Stufe b) erhaltenen Hybrids hervorgegangenen Homokaryen einer ausreichenden Anzahl von Rückkreuzungen mit den aus dem Stamm X hervorgegangenen Homokaryen unterwirft, um einen ausreichenden Anteil des Genoms von X in das entstehende Hybrid einzubauen, wobei man wenigstens eine der Rückkreuzungen mit X2 durchführt.

19. Verfahren gemäß Anspruch 18, in dem man die Kreuzung und die etwaigen Rückkreuzungen mit dem Homokaryon X1 durchführt, mit Ausnahme der letzten Rückkreuzung, welche mit dem Homokaryon X2 durchgeführt wird, wobei am Ende jeder Kreuzungs- und Rückkreuzungsstufe, ausgenommen gegebenenfalls am Ende der vorletzten Rückkreuzung, die tetrasporen Homokaryen ausgewählt werden.

20. Verfahren zum Erhalt eines heterokaryotischen Myceliums gemäß wenigstens einem der Ansprüche 7 bis 9, welches für wenigstens ein rezessives Allel, das in dem Stamm Z nicht vorkommt, homozygot ist, in dem man
a) einen Stamm X des bisporen Agaricus bisporus, welcher für das Allel homozygot ist, mit einem Stamm Y von Agaricus mit tetrasporem Charakter kreuzt, um ein Hybrid XY zu erhalten,
b) ein Homokaryon H von XY auswählt, welches das rezessive Allel und gleichzeitig das tetraspore Merkmal aufweist,
c) zwei interkompatible Homokaryen Z1 und Z2 des Stammes Z auswählt,
d) eine Kreuzung zwischen dem Homokaryon H und Z1 und anschließend gegebenenfalls eine Rückkreuzung mit Z1 durchführt, wobei in jeder Stufe ein Homokaryon eines erhaltenen Hybrids mit tetrasporem Charakter ausgewählt wird, welches das rezessive Allel trägt,
e) eine Kreuzung zwischen dem Homokaryon H und Z2 und anschließend gegebenenfalls wenigstens eine Rückkreuzung mit Z2 durchführt, wobei in jeder Stufe ein Homokaryon eines erhaltenen Hybrids ausgewählt wird, dessen Fruchtentwicklungen Sporen mit hauptsächlich tetrasporem Charakter aufweisen, wobei das Homokaryon das rezessive Allel trägt,
f) und mit Hilfe der Homokaryen, welche das rezessive Allel tragen, die jeweils in d) und e) erhaltenen Kreuzungsprodukte untereinander rückkreuzt.

21. Verfahren gemäß Anspruch 20, in dem die Stufe f) mit Hilfe zweier Homokaryen, von denen wenigstens eines tetrasporen Charakter aufweist, durchgeführt wird.

22. Verfahren gemäß Anspruch 20, in dem die Stufe f) mit Hilfe zweier Homokaryen, welche bisporen Charakter aufweisen, durchgeführt wird.

23. Carpophor, welches aus den Fruchtentwicklungen eines heterokaryotischen Myceliums gemäß wenigstens einem der Ansprüche 1, 3, 5 und 7 bis 10 erhalten werden kann.

## Claims

1. Heterokaryotic mycelium which is more than 75% isogenic with a given bisporous strain X of Agaricus bisporus,said mycelium having the trait of being tetrasporous, that is to say giving fructifications whose spores are mostly homokaryotic.

2. Homokaryon which can be obtained from spores originating from fructifications of a heterokaryotic mycelium according to Claim 1, said homokaryon bearing the trait of being tetrasporous.

3. Heterokaryotic mycelium according to Claim 1 having, for the isogenic part, at least some of the heterozygocity which is characteristic of the said strain X.

4. Homokaryon which can be obtained from spores originating from fructifications of a heterokaryotic mycelium according to Claim 3, the homokaryon bearing the trait of being tetrasporous.

5. Heterokaryotic mycelium which is more than 50% isogenic with a given bisporous strain X of Agaricus bisporus,said mycelium having the trait of being tetrasporous, that is to say giving fructifications whose spores are mostly homokaryotic, and having, for the isogenic part, at least some of the heterozygocity which is characteristic of the said strain X.

6. Homokaryon which can be obtained from spores originating from fructifications of a heterokaryotic mycelium according to Claim 5, said homokaryon bearing the trait of being tetrasporous.

7. Heterokaryotic mycelium which is more than 50% isogenic with a given bisporous strain Z of Agaricus bisporus,said mycelium being homozygous for the trait of being tetrasporous and a recessive trait which is absent in the said strain Z.

8. Heterokaryotic mycelium according to Claim 7 which is more than 75% isogenic with the said strain Z.

9. Heterokaryotic mycelium according to Claim 7 or 8, having the trait of being tetrasporous.

10. Heterokaryotic mycelium which is over 50% isogenic with a given bisporous strain Z of Agaricus bisporus, the mycelium having the trait of being tetrasporous and comprising a dominant allele and a recessive allele of a trait where the recessive allele is absent in strain Z.

11. Homokaryon which can be obtained from spores originating from fructifications of a heterokaryotic mycelium as defined in Claim 9 or 10.

12. Homokaryon according to Claim 11, bearing the recessive trait and the trait of being tetrasporous.

13. Homokaryon according to Claim 11, bearing the recessive trait and the character of being bisporous.

14. Use of at least one homokaryon as defined in Claim 2, 4 or 6 for obtaining hybrids derived from the said strain X.

15. Use of at least one homokaryon as defined in Claim 12 or 13 for obtaining hybrids derived from strain Z, the hybrids being homozygous for a recessive trait which is absent in strain Z.

16. Use of a heterokaryotic mycelium as defined in any of Claims 1, 3, 5, 7, 9 and 10 for obtaining carpophores.

17. Method of obtaining a heterokaryotic mycelium as defined in Claim 1 or 3 in which:
- a first homokaryon originating from strain X is crossed with a second homokaryon originating from a strain Y of Agaricus to obtain a hybrid XY, the strain Y being interfertile with Agaricus bisporus and having the trait of being tetrasporous,
- and the homokaryons originating from homokaryotic spores of the hybrid XY are backcrossed a sufficient time with the first homokaryon in order to incorporate a sufficient part of the genome X into the final hybrid, while selecting at each step of the backcrosses the hybrids which have the trait of being tetrasporous.

18. Method of obtaining a heterokaryotic mycelium as defined in Claim 5, in which:
a) two homokaryons X1 and X2 which are compatible with each other are selected from strain X,
b) homokaryon X1 is crossed with a homokaryon originating from a strain Y of Agaricus which has the trait of being tetrasporous and is interfertile with Agaricus bisporus, in order to obtain a hybrid X1Y,
c) homokaryons originating from homokaryotic spores of a hybrid obtained in step b) are backcrossed sufficiently often with the homokaryons originating from strain X in order to incorporate a sufficient proportion of the genome of X into the final hybrid, and in which at least one of the backcrosses with X2 is carried out.

19. Method according to Claim 18 in which the crossing and optional backcrosses are performed with homokaryon X1, with the exception of the last backcross, which is carried out with homokaryon X2, it being understood that at the conclusion of each crossing and backcrossing step, optionally excepted for the conclusion of the penultimate backcross, homokaryons which have the trait of being tetrasporous are selected.

20. Method of obtaining a heterokaryotic mycelium as defined in any of Claims 7 to 9, which is homozygous for at least one allele of a recessive trait which is absent in strain Z, in which:
a) a bisporous strain X of Agaricus bisporus which is homozygous for the allele is crossed with a strain Y of Agaricus which has the trait of being tetrasporous to obtain a hybrid XY,
b) a homokaryon H of XY is chosen which bears at the same time the recessive allele and the trait of being tetrasporous,
c) two homokaryons Z1 and Z2 of strain Z which are compatible with each other are chosen,
d) the homokaryon H and Z1 are crossed, optionally followed by at least one backcross with Z1, while selecting at each step a homokaryon of a hybrid obtained which has the trait of being tetrasporous, the homokaryon bearing the recessive allele,
e) the homokaryon H and Z2 are crossed, optionally followed by at least one backcross with Z2, while selecting at each step a homokaryon of a hybrid obtained whose fructifications have spores most of which have the trait of being tetrasporous, the homokaryon bearing the recessive allele,
f) the products of the crosses obtained in d) and e), respectively, are again hybridized with each other with the aid of homokaryons bearing the recessive allele.

21. Method according to Claim 20 in which step f) is carried out with the aid of two homokaryons of which at least one bears the trait of being tetrasporous.

22. Method according to Claim 20 in which step f) is carried out with the aid of two homokaryons bearing the trait of being bisporous.

23. Carpophore which can be obtained from the fructification of a heterokaryotic mycelium as defined in any of Claims 1, 3, 5 and 7 to 10.
